(19) **Europäisches Patentamt / European Patent Office / Office européen des brevets**

(11) **EP 4 659 673 A1**

(12) **EUROPEAN PATENT APPLICATION**
published in accordance with Art. 153(4) EPC

(43) Date of publication:
**10.12.2025 Bulletin 2025/50**

(21) Application number: **24770453.9**

(22) Date of filing: **21.02.2024**

(51) International Patent Classification (IPC):
*A61B 5/33* (2021.01)    *A61B 5/02* (2006.01)
*A61B 5/352* (2021.01)    *A61B 5/353* (2021.01)
*A61B 5/361* (2021.01)

(52) Cooperative Patent Classification (CPC):
A61B 5/02; A61B 5/33; A61B 5/352; A61B 5/353;
A61B 5/361

(86) International application number:
**PCT/JP2024/006329**

(87) International publication number:
**WO 2024/190340 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.03.2023   JP 2023040870**

(71) Applicants:
• **Omron Corporation**
**Kyoto-shi, Kyoto 600-8530 (JP)**
• **Omron Healthcare Co., Ltd.**
**Muko-shi, Kyoto 617-0002 (JP)**

(72) Inventors:
• **KUBO, Mitsuaki**
**Kyoto-shi, Kyoto 600-8530 (JP)**
• **KOIZUMI, Masayuki**
**Kyoto-shi, Kyoto 600-8530 (JP)**
• **WANG, Danni**
**Kyoto-shi, Kyoto 600-8530 (JP)**
• **KIMURA ISHIDA, Yui**
**Kyoto-shi, Kyoto 600-8530 (JP)**
• **FUJII, Kenji**
**Muko-shi, Kyoto 617-0002 (JP)**
• **KAWABATA, Yasuhiro**
**Muko-shi, Kyoto 617-0002 (JP)**

(74) Representative: **Isarpatent**
**Patent- und Rechtsanwälte**
**Barth Hassa Peckmann & Partner mbB**
**Friedrichstraße 31**
**80801 München (DE)**

(54) **BIOLOGICAL SIGNAL MEASUREMENT DEVICE AND METHOD FOR CONTROLLING BIOLOGICAL SIGNAL MEASUREMENT DEVICE**

(57)    A biological signal measurement device that is worn by a user and performs constant measurement of a biological signal includes an ECG sensor that measures an ECG signal on a limb of the user, a pulse wave sensor that measures a pulse wave of the user, and an information processing unit that acquires information regarding a heart rate variability and information regarding an abnormality of an electrocardiographic waveform, based on time-series data of the ECG signal obtained by the ECG sensor and time-series data of the pulse wave obtained by the pulse wave sensor.

FIG. 1

EP 4 659 673 A1

**Description**

TECHNICAL FIELD

The present invention relates to a biological signal measurement device for constantly measuring a biological signal.

BACKGROUND ART

Atrial fibrillation (AF) is a type of arrhythmia, and is a disease in which the atria fibrillate rapidly due to abnormal electrical signals generated from sites other than the sinoatrial node, and the pulse (heartbeat interval) becomes irregular. Atrial fibrillation not only causes palpitations and shortness of breath, which can interfere with daily life, but also has the potential to cause serious diseases such as stroke and heart failure. Therefore, early detection and appropriate treatment of atrial fibrillation are desired.

Diagnosis of atrial fibrillation is usually performed by evaluating disturbance of heartbeat intervals by electrocardiogram (ECG). In addition, when the onset of atrial fibrillation is unpredictable, as in the case of paroxysmal atrial fibrillation, it is difficult to detect and diagnose atrial fibrillation with a short-term electrocardiogram examination. Therefore, a long-term electrocardiogram using a 24-hour Holter electrocardiograph is generally used. The 24-hour Holter electrocardiograph is a portable electrocardiograph that is capable of constantly measuring and recording an electrocardiogram in daily life by attaching electrodes to several portions of the chest with a conductive adhesive gel. Patent Document 1 proposes an idea of plotting R-R intervals (intervals between an R wave and a subsequent R wave in an electrocardiogram) on a two-dimensional scatter plot, based on long-term electrocardiogram data measured by a 24-hour Holter electrocardiograph, and determining the presence or absence of paroxysmal atrial fibrillation from features observed in the distribution.

CITATION LIST

PATENT LITERATURE

Patent Document 1: JP 2004-16248 A

SUMMARY OF INVENTION

TECHNICAL PROBLEM

The use of a long-term electrocardiogram is essential for the accurate diagnosis of paroxysmal atrial fibrillation. In addition, recently, an index called AF burden (atrial fibrillation burden) is gaining recognition, and the importance of evaluating the longest duration of atrial fibrillation, the number of episodes per day, the cumulative time of atrial fibrillation, and the like from a long-term electrocardiogram is increasing.

Therefore, the present inventors have been developing a type of electrocardiograph (hereinafter, referred to as a "limb-type electrocardiograph") that is worn on a body part such as the arm, wrist, or leg of a user, in order to facilitate long-term electrocardiogram measurement. However, the limb-type electrocardiograph has a problem in that it is more susceptible to myoelectric noise compared to the Holter electrocardiograph. The electrocardiogram is based on the principle of measuring weak electrical signals (ECG signals) generated during heartbeats by electrodes attached to the skin surface, and therefore, myoelectric signals generated by body motion become noise. The Holter electrocardiograph is hardly affected by body motion because the electrodes are attached mainly to the chest, whereas the parts (arm, wrist, leg, and the like) to which the limb-type electrocardiograph is attached move significantly, and therefore, the mixing of myoelectric noise during activities, such as in the daytime, is unavoidable. Furthermore, since the distance from the heart to the electrode is longer in the limb-type electrocardiograph than in the Holter electrocardiograph, the potential of the ECG signal attenuates more significantly, and the SN ratio is more likely to deteriorate. Therefore, even when an electrocardiogram can be measured for 24 hours by the limb-type electrocardiograph, data obtained during the daytime (active hours) may not be mostly usable for diagnosis of atrial fibrillation.

On the other hand, products such as Apple Watch (trade name) have appeared that claim that atrial fibrillation can be detected by a photoplethysmography (PPG) sensor. The PPG sensor is based on the principle of measuring pulse waves by optically measuring changes in blood flow volume, and therefore may be more advantageous than an electrocardiograph in that it is not affected by myoelectric noise. However, strictly speaking, it is not possible to accurately determine atrial fibrillation from pulse wave data obtained by the PPG sensor. This is because, for the diagnosis of atrial fibrillation, it is essential to determine whether the atria are functioning in accordance with electrical signals from the sinoatrial node or in accordance with other abnormal electrical signals, and for the determination, it is necessary to confirm whether a P wave (a wave indicating atrial excitation) is present or absent. The pulse wave data does not include information regarding P waves. Therefore, although it is possible to know from the pulse wave data that arrhythmia (tachycardia or irregular pulse) has occurred, it is not possible to distinguish whether the arrhythmia is caused by atrial fibrillation or another disease (for example, premature contraction).

Although atrial fibrillation has been described as an example, similar problems occur in the case of cardiovascular diseases such as ischemic heart disease and premature ventricular contraction. That is, with the limb-type electrocardiograph, stable measurement and diagnosis are difficult due to the influence of the myoelectric noise, and accurate determination of cardiovascular diseases is not possible because an electrocardiographic waveform cannot be captured from the pulse wave data.

The present invention has been made in view of the above circumstances, and an object thereof is to provide a technique capable of measuring data useful for diagnosis of a cardiovascular disease such as atrial fibrillation in daily life.

SOLUTION TO PROBLEM

The present disclosure includes a biological signal measurement device configured to be worn by a user and to perform constant measurement of a biological signal, the biological signal measurement device including an ECG sensor configured to measure an ECG signal on a limb of the user, a pulse wave sensor configured to measure a pulse wave of the user, and an information processing unit configured to acquire information regarding a heart rate variability and information regarding an abnormality of an electrocardiographic waveform, based on time-series data of the ECG signal obtained by the ECG sensor and time-series data of the pulse wave obtained by the pulse wave sensor.

The information regarding the abnormality of the electrocardiographic waveform may include information regarding a presence or absence of a P wave. The information processing unit may detect an occurrence of atrial fibrillation, based on the information regarding the heart rate variability and the information regarding the presence or absence of the P wave that are acquired from the time-series data of the ECG signal and the time-series data of the pulse wave that are constantly measured.

The information processing unit may generate an index related to atrial fibrillation, based on a detection result of atrial fibrillation during a predetermined measurement period, and may record or output the index.

The index related to atrial fibrillation may include at least one of a longest atrial fibrillation duration during the predetermined measurement period, the number of occurrences of atrial fibrillation during the predetermined measurement period, or a cumulative time of atrial fibrillation during the predetermined measurement period.

The information processing unit may acquire the information regarding the abnormality of the electrocardiographic waveform from the time-series data of the ECG signal, and acquire the information regarding the heart rate variability from the time-series data of the pulse wave.

The information processing unit may include an ECG signal quality determination unit configured to determine whether signal quality of the ECG signal is good or poor, and a pulse wave signal quality determination unit configured to determine whether signal quality of the pulse wave is good or poor, and may acquire the information regarding the heart rate variability from the time-series data of the ECG signal during a period in which the signal quality of the ECG signal is determined to be good, and may acquire the information regarding the heart rate variability from the time-series data of the pulse wave during a period in which the signal quality of the ECG signal is determined to be poor.

The biological signal measurement device may include a dry electrode, and a member configured to fix the electrode in a state in which the electrode is pressed against the limb of the user, in which the ECG sensor may measure the ECG signal by the electrode fixed by the member.

The pulse wave sensor may be a photoplethysmography (PPG) sensor.

The present disclosure includes a method for controlling a biological signal measurement device configured to be worn by a user and to perform constant measurement of a biological signal, the method including measuring an ECG signal on a limb of the user by an ECG sensor, measuring a pulse wave of the user by a pulse wave sensor, and acquiring information regarding a heart rate variability and information regarding an abnormality of an electrocardiographic waveform, based on time-series data of the ECG signal obtained by the ECG sensor and time-series data of the pulse wave obtained by the pulse wave sensor.

The present invention may be regarded as a biological signal measurement device including at least a part of the above configuration, or may be regarded as an electrocardiogram measurement device that measures an ECG signal as a biological signal. Alternatively, the present invention may be regarded as an atrial fibrillation detection device, an atrial fibrillation recording device, or an atrial fibrillation monitoring device. Further, the present invention can be regarded as a control method including at least a part of the above processing, or a program for implementing such a method and a nontransitory recording medium on which the program is recorded. Note that the present invention can be configured by combining each of the above-described configurations and processes to the extent possible.

ADVANTAGEOUS EFFECTS OF INVENTION

According to the present invention, it is possible to measure data useful for diagnosis of a cardiovascular disease such as atrial fibrillation in daily life.

BRIEF DESCRIPTION OF THE DRAWINGS

[FIG. 1] FIG. 1 is a diagram illustrating a state in which a biological signal measurement device is worn on an upper arm.
[FIG. 2] FIG. 2 is a plan view of the biological signal measurement device.
[FIG. 3] FIG. 3 is a perspective view of the biological signal measurement device.
[FIG. 4] FIG. 4 is a block diagram illustrating a functional configuration of a biological signal measurement device in a first embodiment.
[FIG. 5] FIG. 5 is a flowchart illustrating a flow of a measurement process in the first embodiment.
[FIG. 6] FIG. 6 is a diagram illustrating an example of an electrode pair and an ECG signal.
[FIG. 7] FIG. 7 is a diagram illustrating a waveform of an ECG signal and heart rate information.
[FIG. 8] FIG. 8 is a diagram illustrating a measurement principle of a PPG signal.
[FIG. 9] FIG. 9 is a diagram illustrating an example of a waveform of a PPG signal.
[FIG. 10] FIG. 10 is a block diagram illustrating a functional configuration of a biological signal measurement device in a second embodiment.
[FIG. 11] FIG. 11 is a flowchart illustrating a flow of a measurement process in the second embodiment.

DESCRIPTION OF EMBODIMENTS

<Application Example>

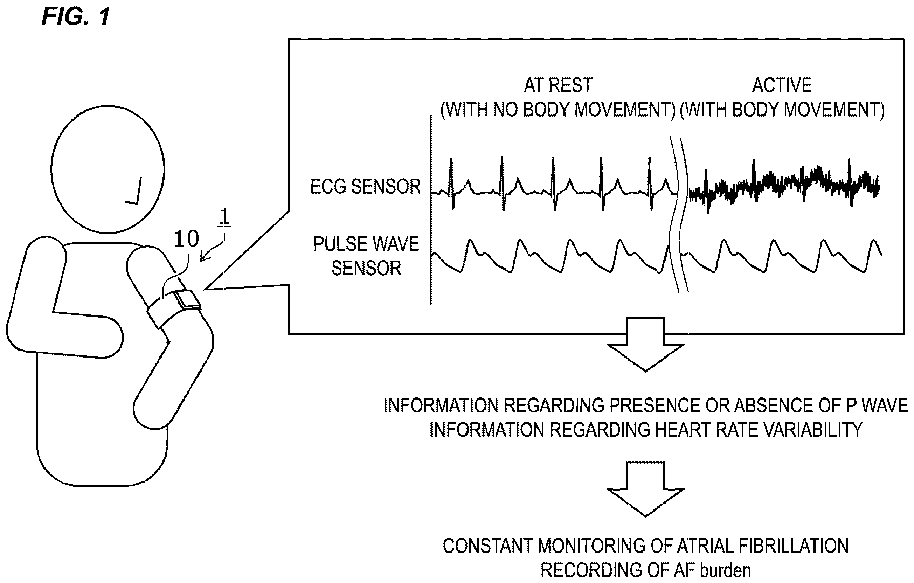

An application example of the present invention will be described with reference to FIG. 1.

A biological signal measurement device 1 is a portable measurement device that is used by being worn on a limb, and is capable of 24-hour monitoring of an ECG signal (electrocardiogram signal) and cardiovascular diseases such as atrial fibrillation. The biological signal measurement device 1 includes an ECG sensor that measures an ECG signal and a pulse wave sensor that measures a pulse wave.

At the time of measurement, a user wears the biological signal measurement device 1 by wrapping a band 10 around a measurement site. Examples of measurement sites where the band 10 can be attached include upper limbs (upper arms, forearms, wrists, hands, and fingers), lower limbs (thighs, lower legs, ankles, feet, and toes), and the like, and a measurement site is appropriately selected depending on the type of biological signal to be measured, a measurement algorithm, and the like.

As described above, a long-term electrocardiogram is required for accurate diagnosis of cardiovascular diseases. However, when body motion is significant, the SN ratio decreases due to mixing of myoelectric noise, and stable measurement and diagnosis become difficult. On the other hand, the measurement data of the pulse wave sensor is less likely to be affected by the body motion, but does not enable accurate determination of cardiovascular diseases.

Therefore, the biological signal measurement device 1 adopts a configuration in which information necessary for diagnosis of cardiovascular diseases (for example, information regarding heart rate variability and information regarding an abnormality of an electrocardiographic waveform) is acquired by combining time-series data of an ECG signal obtained by the ECG sensor and time-series data of a pulse wave obtained by the pulse wave sensor. As one method, "information regarding an abnormality of an electrocardiographic waveform may be acquired from time-series data of an ECG signal, and information regarding heart rate variability may be acquired from time-series data of a pulse wave". Alternatively, as another method, dynamic switching may be adopted such that "information regarding an abnormality of an electrocardiographic waveform is acquired from time-series data of an ECG signal, and information regarding heart rate variability is acquired from time-series data of an ECG signal when quality of the ECG signal is good (when body motion is slight and the SN ratio is high), and is acquired from time-series data of a pulse wave when the quality is poor (when body motion is significant and the SN ratio of the ECG signal is low)". Alternatively, as another method, a learned model that has been machinelearned so as to "output both information regarding heart rate variability and information regarding an abnormality of an electrocardiographic waveform when both time-series data of an ECG signal and time-series data of a pulse wave are input" may be used. In this way, by complementarily using the measurement data of the ECG sensor and the measurement data of the pulse wave sensor, constant monitoring of highly reliable information necessary for diagnosis of cardiovascular diseases and recording of an index related to a cardiovascular disease (for example, AF burden that is an index related to atrial fibrillation) can be performed regardless of the presence or absence of body motion.

When the biological signal measurement device 1 performs detection of atrial fibrillation, it may acquire information regarding the presence or absence of a P wave as the information regarding an abnormality of an electrocardiographic waveform from the time-series data of the ECG signal. For example, when performing detection of an ischemic heart disease, it may acquire an increase or decrease in ST, an abnormality of a Q wave, or the like as the information regarding an abnormality of an electrocardiographic waveform from the time-series data of the ECG signal. In addition, for example, when performing detection of a premature ventricular contraction, it may acquire an abnormality of a QRS wave as the information regarding an abnormality of an electrocardiographic waveform from the time-series data of the ECG signal.

Hereinafter, as an embodiment of the present invention, a specific configuration example in a case where the present invention is applied to diagnosis of atrial fibrillation will be described.

<First Embodiment>

(Device Configuration)

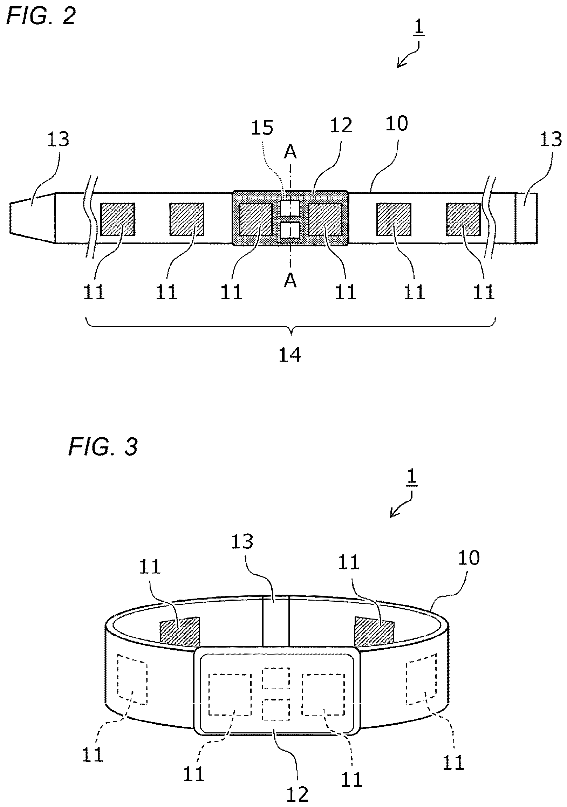

An embodiment of the present invention will be described with reference to FIGS. 1 to 3. FIG. 1 is a diagram illustrating a state in which the biological signal measurement device 1 is worn on an upper arm, FIG. 2 is a plan view of the biological signal measurement device 1, and FIG. 3 is a perspective view of the biological signal measurement device 1.

The biological signal measurement device 1 of the present embodiment is an upper arm electrocardiographic device that is worn on an upper arm (preferably, a left upper arm close to the heart) of a user and is used to measure an electrocardiogram (ECG) signal as a biological signal.

The biological signal measurement device 1 includes, as main components, a band 10, a plurality of electrodes 11 fixed to the band 10, and a control body 12 fixed to the band 10. An ECG sensor 14 is configured by the plurality of electrodes 11 and a processing circuit built in the control body 12. In addition, a pulse wave sensor 15 is built in the control body 12.

The band 10 is a member (fixing member) for fixing the electrodes 11 in a state in which the electrodes are pressed against the living body. In the present embodiment, a belt-shaped band 10 made of a flexible and pliable material (for example, chemical fiber, silicon, leather, or the like) is used. A fixing mechanism 13 is provided at a longitudinal end portion of the band 10. As illustrated in FIGS. 1 and 3, the biological signal measurement device 1 can be worn on the upper arm by forming the band 10 into a loop shape and fastening the band with the fixing mechanism 13. The fixing mechanism 13 may be any mechanism such as a hook-and-loop fastener, a hook, a connector, a button, or a magnet.

The plurality of electrodes 11 (also referred to as an electrode array) are embedded and fixed in the band 10 such that a contact surface with the living body is exposed to an inner side (living body side) of the band 10. The plurality of electrodes 11 are arranged in a line at equal intervals in the longitudinal direction of the band 10. Thus, when the band 10 is wrapped around the arm, the electrodes 11 come into contact with different circumferential positions of the arm. The number of electrodes 11 is not particularly limited and can be appropriately designed. At least two electrodes 11 (a pair of electrodes) may be provided to measure an ECG signal, and three or more electrodes 11 may be provided to increase the reliability and robustness of measurement. In the present embodiment, a configuration in which six electrodes 11 (three electrode pairs) are provided is adopted.

The electrode 11 is a dry-type metal electrode. The wet-type electrode (gel electrode or the like) has problems such as a possibility of causing skin rash or itching when attached for a long time, and low durability and maintainability, whereas the dry-type electrode 11 does not have such problems. The biological signal measurement device 1 of the present embodiment is assumed to be worn continuously for a long time and to monitor the ECG signal for 24 hours, and thus the electrodes 11 are preferably dry-type electrodes.

The control body 12 is a processing unit that performs control and signal processing of the biological signal measurement device 1. The control body 12 has a structure in which a processor, a memory, a battery, and other circuits are mounted inside a case made of resin or metal, for example. The control body 12 may be provided with a physical switch and a display. Although not illustrated, the control body 12 and the plurality of electrodes 11 are connected via signal lines.

(Functional Configuration)

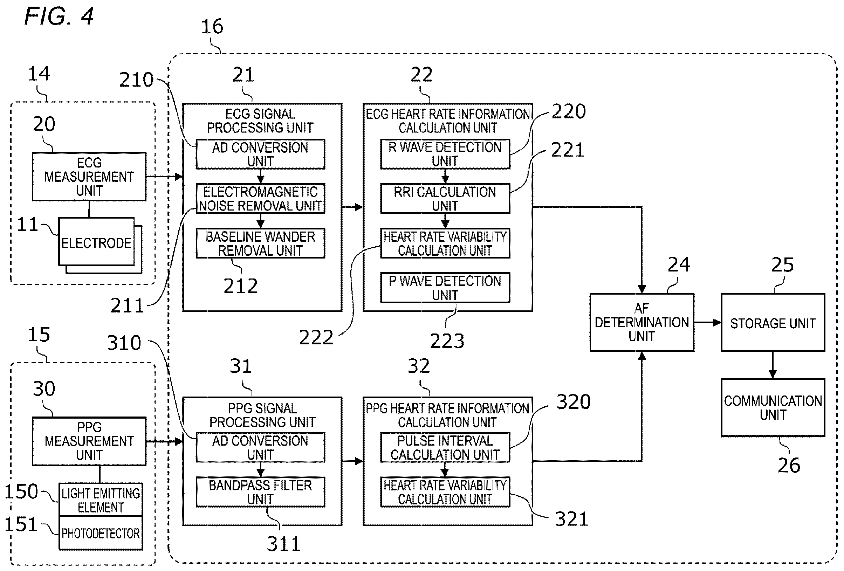

FIG. 4 is a block diagram illustrating a functional configuration of the biological signal measurement device 1.

The biological signal measurement device 1 includes an ECG sensor 14, a pulse wave sensor 15, and an information processing unit 16. The ECG sensor 14 is a sensor that measures an ECG signal, and generally includes a plurality of electrodes 11 and an ECG measurement unit 20. The ECG measurement unit 20 is a circuit that amplifies a potential difference between an electrode pair by a differential amplifier and outputs the amplified potential difference as an ECG signal. The pulse wave sensor 15 is a sensor that measures a pulse wave, and a PPG sensor is used in the present embodiment. The pulse wave sensor 15 generally includes a light emitting element 150 for emitting light, a photodetector 151 for detecting reflected light and photoelectrically converting the reflected light, and a PPG measurement unit 30 for outputting the intensity of the reflected light as a PPG signal. An LED or the like that emits visible light or infrared light is used as the light emitting element 150, and a photodiode, a phototransistor, or the like is used as the photodetector 151.

The information processing unit 16 includes an ECG signal processing unit 21 and an ECG heart rate information calculation unit 22, as components related to the measurement of the ECG signal. The ECG signal processing unit 21 is a part that performs AD conversion and filtering processing of the ECG signal, and includes an AD conversion unit 210 that performs AD conversion of the ECG signal, an electromagnetic noise removal unit 211 that removes electromagnetic noise from the ECG signal to increase an SN ratio, and a baseline wander removal unit 212 that removes baseline wander (low-frequency wander) of the ECG signal. The ECG heart rate information calculation unit 22 is a part that extracts various types of heart rate information from the ECG signal, and includes an R wave detection unit 220, an RRI calculation unit 221, a heart rate variability calculation unit 222, and a P wave detection unit 223.

The information processing unit 16 includes a PPG signal processing unit 31 and a PPG heart rate information calculation unit 32, as components related to the measurement of the PPG signal. The PPG signal processing unit 31 is a part that performs AD conversion and filtering processing of the PPG signal, and includes an AD conversion unit 310 that performs AD conversion of the PPG signal, and a bandpass filter unit 311 that removes noise from the PPG signal to increase an SN ratio. The PPG heart rate information calculation unit 32 is a part that extracts various types of heart rate information from the PPG signal, and includes a pulse interval calculation unit 320, and a heart rate variability calculation unit 321.

The information processing unit 16 further includes an AF determination unit 24, a storage unit 25, and a communication unit 26. The AF determination unit 24 is a part that detects an occurrence of atrial fibrillation (AF), based on information obtained from the ECG signal and information obtained from the PPG signal, and calculates an index related to AF. The storage unit 25 is a nonvolatile memory that stores measured or calculated data. The communication unit 26 is a part that performs wireless data communication with an external device (for example, a smartphone of a user, another health device, a home server, or the like). Although not illustrated, the information processing unit 16 may include an operation unit including a physical button, a touch panel display, and the like.

(Measurement Process)

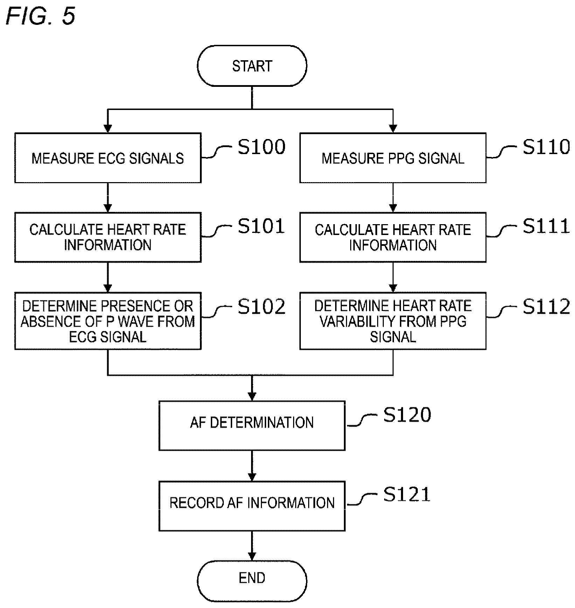

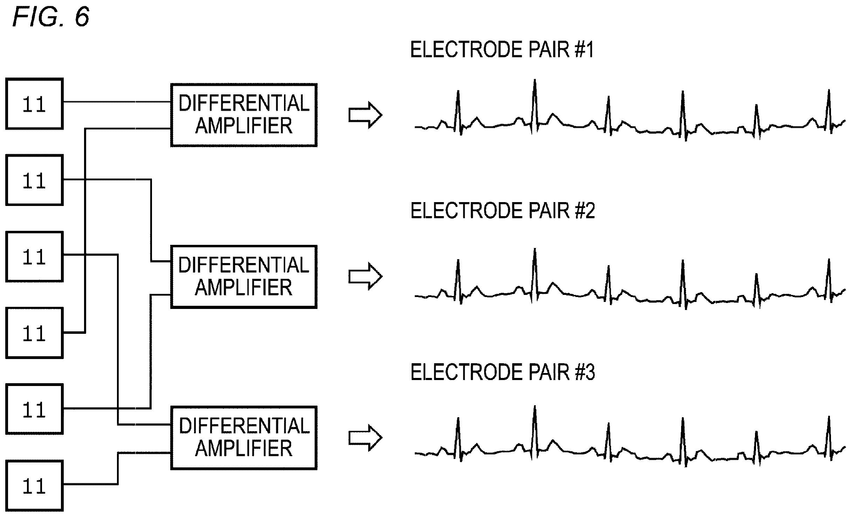

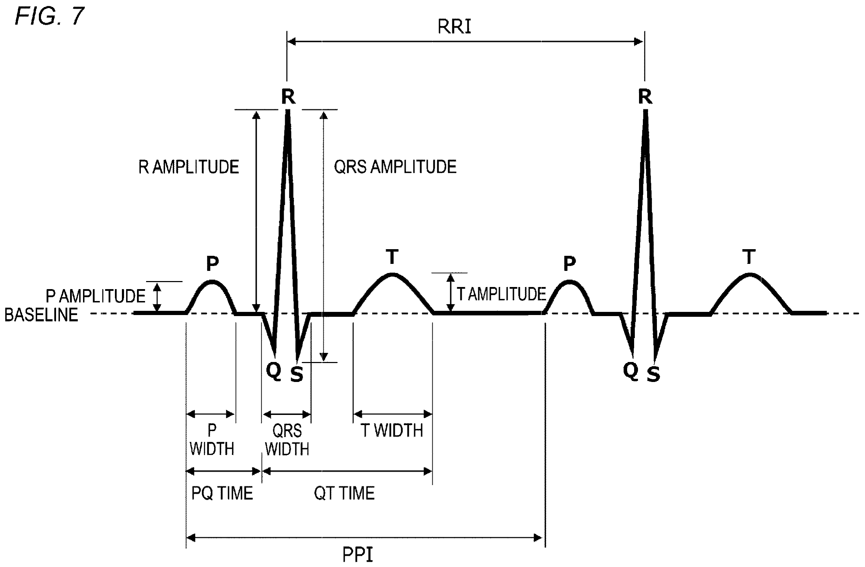

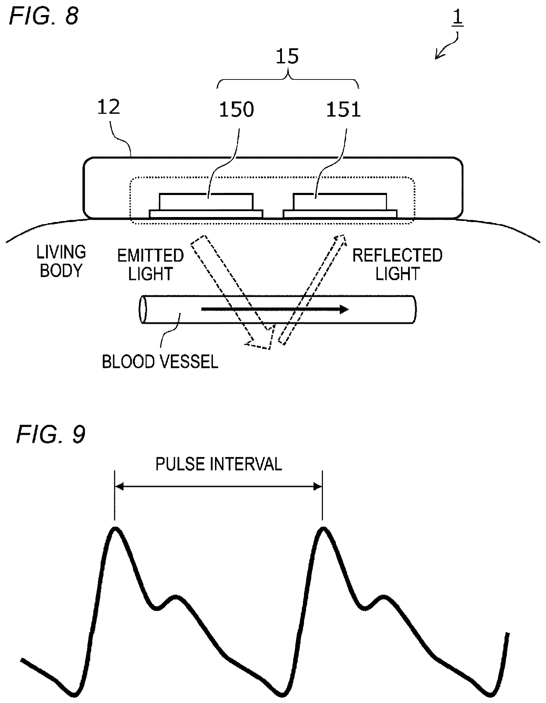

A measurement process by the biological signal measurement device 1 will be described with reference to FIGS. 5 to 9. FIG. 5 is a flowchart illustrating a flow of a measurement process, FIG. 6 is a diagram illustrating an example of an electrode pair and an ECG signal, FIG. 7 is a diagram illustrating a waveform of an ECG signal and heart rate information, FIG. 8 is a diagram illustrating a measurement principle of a PPG signal, and FIG. 9 is a diagram illustrating an example of a waveform of a PPG signal.

When the user wraps the band 10 around the upper arm, fastens the band 10 with the fixing mechanism 13, and then performs an operation of instructing the start of measurement, the processor of the control body 12 starts the measurement process of FIG. 5. In the biological signal measurement device 1, an ECG signal measurement process (steps S100 to S102) and a PPG signal measurement process (steps S110 to S112) are executed in parallel, and an AF determination process (steps S120 and S121) is executed using information acquired by these processes. The three processes will be described in the order below.

(1) ECG Signal Measurement Process

In step S100, the ECG measurement unit 20 measures ECG signals of three channels using three electrode pairs. Specifically, the electrode pair (two electrodes 11) to be used for measurement is selected, and the potential difference between the selected electrode pair is amplified by the differential amplifier and taken in as an ECG signal (analog voltage signal). By sequentially switching the electrode pairs to be selected, ECG signals of three channels are taken in.

In the present embodiment, as illustrated in FIG. 6, three electrode pairs are set such that two electrodes 11 that are just opposite to each other when the band 10 is wrapped around the upper arm are paired. This is because a larger potential difference (i.e., an ECG signal having a higher SN ratio) can be measured when the two electrodes 11 forming a pair are separated from each other. However, the method of setting the electrode pairs is not limited thereto. For example, a multiplexer may be used to freely switch the combination of the electrodes 11 to be paired. In this case, ECG signals of four or more channels can be measured from the six electrodes 11.

The ECG signal processing unit 21 performs AD conversion of the taken-in ECG signal, and removes electromagnetic noise and baseline wander by digital signal processing. A known noise reduction method such as a bandpass filter, a notch filter, or a moving average can be used to remove electromagnetic noise and baseline wander.

In step S101, the ECG heart rate information calculation unit 22 analyzes the taken-in ECG signal and calculates various types of heart rate information. The ECG heart rate information calculation unit 22 performs the processing of step S101 when time-series data (waveform data) of an ECG signal for a predetermined unit time is taken in from the ECG signal processing unit 21. The unit time may be set to, for example, a time of about 10 seconds to 600 seconds, and is set to 60 seconds in the present embodiment.

As schematically illustrated in FIG. 7, the waveform of one heart rate of the ECG signal mainly includes a P wave, a QRS wave, and a T wave. Note that the QRS wave indicates a waveform obtained by combining an R wave, which is an upward peak, and a downward Q wave and a downward S wave that appear before and after the R wave. In a normal heart, an electrical stimulus generated at the sinoatrial node is transmitted from the atrium to the ventricle, and excitation (contraction) of the atrium and excitation (contraction) of the ventricle occur in sequence, thereby pumping blood. The P wave is a waveform corresponding to the excitation of the atrium, the QRS wave is a waveform corresponding to the excitation of the ventricle, and the T wave is a waveform corresponding to the process of recovery of the ventricle from the excitation. In the case of an average adult, the heart rate is about 60 to 80 bpm, and thus, about 60 to 80 heart rate waveforms are included in the time-series data of the ECG signal for a unit time (60 seconds).

Examples of the heart rate information include an R amplitude (height of R wave from the baseline), a QRS amplitude (defined by, for example, the sum of the height of R wave from the baseline and the depth of S wave from the baseline), a P amplitude (height of P wave from the baseline), a T amplitude (height of T wave from the baseline), a P width (time from start of P wave to end of P wave), a QRS width (time from start of Q wave to end of S wave), a T width (time from start of T wave to end of T wave), a PQ time (time from start of P wave to start of Q wave), a QT time (time from start of Q wave to end of T wave), an RRI (RR interval; time from peak of R wave to peak of next R wave), a PPI (PP interval; time from start of P wave to start of next P wave), a heart rate variability (temporal variation in RRI and PPI), and the like. It is not necessary to calculate all of the heart rate information, and only necessary heart rate information may be calculated. In addition, other heart rate information may be calculated.

For example, in the present embodiment, the R wave detection unit 220 detects the R wave of each heartbeat from the time-series data of the ECG signal, and calculates the R amplitude, the QRS width, and the like. Then, the RRI calculation unit 221 calculates the RRI. When the heartbeat is normal, the RRI is substantially constant (no heart rate variability), but when atrial fibrillation or other arrhythmia occurs, symptoms such as a narrowing of the RRI or an irregular change in the RRI are observed. In addition, the P wave detection unit 223 detects the P wave of each heartbeat from the time-series data of the ECG signal, and calculates the P amplitude, the P width, and the like.

The detection of the P wave can be performed, for example, as follows. The P wave normally precedes the R wave by about 0.1 to 0.2 seconds. Therefore, the P wave detection unit 223 sets, as a P wave candidate point cP, an undulation that appears prior to the R wave with reference to the time of the R wave detected by the R wave detection unit 220, and acquires the intensity of the candidate point cP. The P wave detection unit 223 recognizes the undulation having the candidate point cP as an apex as the P wave when the intensity of the candidate point cP is equal to or greater than a predetermined threshold value Th1, and determines that the P wave cannot be detected when the intensity is less than the threshold value Th1. The threshold value Th1 can be set arbitrarily, but may be set to a value of about 2.5 to 5% of the R amplitude or the QRS amplitude, for example.

In step S102, the AF determination unit 24 determines the presence or absence of a P wave. For example, it may be determined that "a P wave is present" when a ratio of the number of heartbeat waveforms in which a P wave is detected by the P wave detection unit 223 to the total number of heartbeat waveforms included in the ECG signal for a unit time is equal to or greater than a predetermined threshold value Th2, and that "a P wave is absent" when the ratio is less than the threshold value Th2. The threshold value Th2 can be set arbitrarily, and is set to 80% in the present embodiment, for example. The determination result here is "information regarding the presence or absence of a P wave".

The method of determining the presence or absence of a P wave is not limited to the above-described method. For example, one representative heartbeat waveform (hereinafter, referred to as "representative waveform") may be acquired from the ECG signal for a unit time, and it may be determined that "a P wave is present" when the intensity (amplitude) of the P wave candidate point cP in the representative waveform is equal to or greater than a predetermined threshold value Th1, and that "a P wave is absent" when the intensity is less than the threshold value Th1. Alternatively, two or more representative waveforms may be acquired from the ECG signal for a unit time, and it may be determined that "a P wave is present" when a ratio of representative waveforms, among the acquired two or more representative waveforms, in which the P wave candidate point cP having an intensity equal to or higher than the threshold value Th1 is detected, is equal to or higher than a threshold value Th2, and that "a P wave is absent" when the ratio is less than the threshold value Th2. Note that the representative waveform may be a heartbeat waveform selected from a plurality of heartbeat waveforms included in the ECG signal for a unit time, a heartbeat waveform synthesized from all or some of the heartbeat waveforms in the ECG signal for a unit time, or a waveform obtained by further processing the selected or synthesized heartbeat waveform.

(2) PPG Signal Measurement Process

In step S110, the PPG measurement unit 30 measures a PPG signal. The PPG signal is taken into the PPG signal processing unit 31, and is subjected to AD conversion by the AD conversion unit 310, and then noise is removed by the bandpass filter unit 311.

FIG. 8 schematically illustrates a measurement principle of a PPG signal by the pulse wave sensor 15. FIG. 8 corresponds to a cross-sectional view taken along line A-A of FIG. 2. The pulse wave sensor 15 is a reflective PPG sensor having the light emitting element 150 and the photodetector 151 arranged in parallel on a rear surface of the control body 12. When the control body 12 is wrapped around the upper arm by the band 10, the pulse wave sensor 15 is placed in a state of being in close contact with the skin surface. A part of the light emitted from the light emitting element 150 is reflected in the living body and detected by the photodetector 151. At this time, since the energy of the light is absorbed by hemoglobin in the blood, the intensity of the reflected light changes according to the blood flow volume. The PPG signal is a signal obtained by capturing a temporal change in the intensity of the reflected light, and indicates a change in the blood flow volume due to the pulse.

In step S111, the PPG heart rate information calculation unit 32 analyzes the taken-in PPG signal and calculates various types of heart rate information. The PPG heart rate information calculation unit 32 performs the processing of step S111 when time-series data (waveform data) of a PPG signal for a predetermined unit time is taken in from the PPG signal processing unit 31. The unit time is set to be the same time as that of the ECG signal (60 seconds in the present embodiment).

FIG. 9 schematically illustrates a PPG signal waveform. Since a pulse is also a phenomenon that occurs in association with the heartbeat of the heart, the peak period of the PPG signal waveform is synchronized with the ECG signal waveform (however, there is a delay depending on the distance from the heart compared to the ECG signal waveform). However, the ECG signal is data obtained by measuring an electrical signal transmitted from the heart, whereas the PPG signal is data obtained by measuring a temporal change in the blood flow volume. Therefore, the PPG signal exhibits a waveform clearly different from that of the ECG signal, and does not include information corresponding to the P wave.

In the present embodiment, the pulse interval calculation unit 320 detects a pulse peak from the PPG signal waveform, and calculates a time between a peak and a subsequent peak, that is, a pulse interval. The pulse interval is information corresponding to the RRI acquired from the ECG signal. In addition, the heart rate variability calculation unit 321 calculates, as an index of heart rate variability, a variation (for example, variance, standard deviation, or the like) in pulse intervals in the PPG signal waveform for a unit time.

In step S112, the AF determination unit 24 determines the presence or absence of heart rate variability. For example, the AF determination unit 24 may determine that "heart rate variability is present" when the variation in pulse intervals is equal to or greater than a predetermined threshold value, and that "heart rate variability is absent" when the variation is less than the threshold value. The threshold value can be set arbitrarily. The determination result here is "information regarding heart rate variability".

(3) AF Determination Process

In step S120, the AF determination unit 24 determines whether atrial fibrillation occurs from the information regarding the presence or absence of a P wave and the information regarding heart rate variability. For example, when the information indicates that "there is no P wave" and that "heart rate variability is present", it is determined that atrial fibrillation has occurred during the unit time indicated by the waveform data. Although not illustrated, at the timing when the occurrence of atrial fibrillation is detected, a user may be notified, or an alert message or the like may be output from the communication unit 26 to an external device.

In step S121, the AF determination unit 24 records the presence or absence of the occurrence of atrial fibrillation in the storage unit 25 together with the information about the measurement time of the waveform. At this time, the waveform data and the heart rate information calculated in steps S101 and S111 may be recorded together. Accordingly, the AF determination results for each unit time (for example, for each 60 seconds) are accumulated in the storage unit 25.

Further, the AF determination unit 24 may generate and record AF burden, which is an index related to atrial fibrillation, at a timing when data for a predetermined measurement period is accumulated, or the like. The AF burden includes at least one of the longest atrial fibrillation duration during the predetermined measurement period, the number of occurrences of atrial fibrillation during the predetermined measurement period, or a cumulative time of atrial fibrillation during the predetermined measurement period. The measurement period may be set to a length of about several hours to several weeks, for example. When the measurement period is set to one day (= 86400 seconds) and the unit time is set to 60 seconds, the AF burden is the sum of 1440 (= 86400 / 60) AF determination results.

The above-described processes enable 24-hour monitoring of atrial fibrillation and automatic recording of AF burden useful for diagnosis of atrial fibrillation. Note that the AF information (AF determination result, AF burden, and the like) recorded in the storage unit 25 is output from the communication unit 26 to an external device.

<Second Embodiment>

In the first embodiment, the information regarding heart rate variability is acquired from a PPG signal that is robust against body motion. However, the information regarding heart rate variability may be acquired from an ECG signal at rest with no body motion. This is because the R wave peak of the ECG signal is sharper than the peak of the PPG signal, and thus the heartbeat interval and the heart rate variability can be calculated more accurately (with smaller timing error) using the ECG signal. Therefore, in the second embodiment, a method of acquiring the information regarding heart rate variability from the ECG signal when the signal quality of the ECG signal is good is adopted.

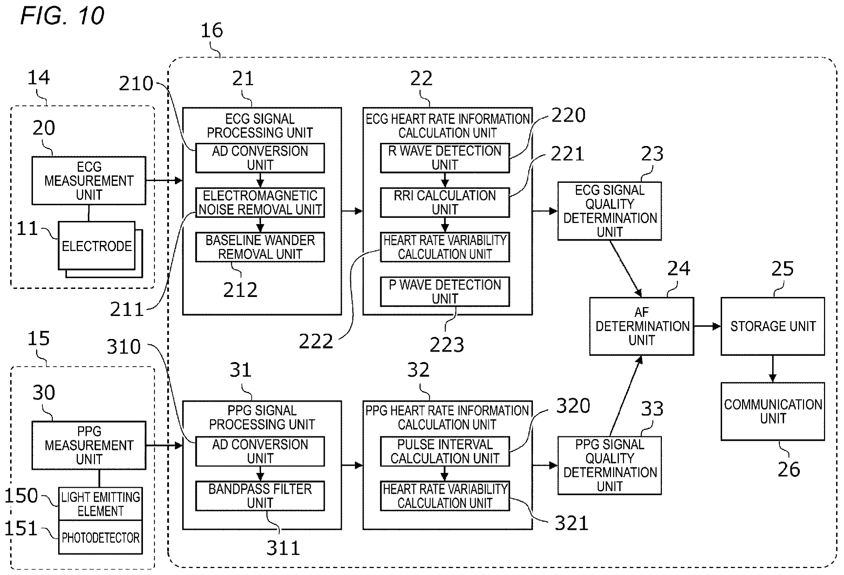

FIG. 10 is a block diagram illustrating a functional configuration of the biological signal measurement device 1 in the second embodiment. The difference from the configuration of the first embodiment (FIG. 4) is that an ECG signal quality determination unit 23 and a PPG signal quality determination unit 33 are provided. The ECG signal quality determination unit 23 is a part that determines whether the quality of the ECG signal is good or poor (that is, whether accurate or reliable data suitable for AF determination or the like can be measured). The PPG signal quality determination unit 33 is a part that determines whether the quality of the PPG signal is good or poor (that is, whether accurate or reliable data suitable for AF determination or the like can be measured).

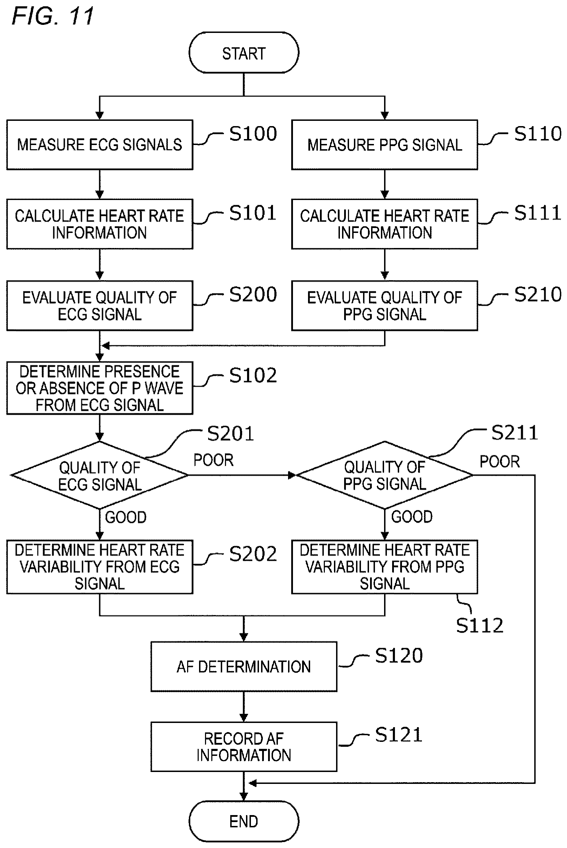

FIG. 11 illustrates a flow of a measurement process by the biological signal measurement device 1 in the second embodiment. The same step numbers are given to the same parts as those in the determination process of the first embodiment (FIG. 5). Hereinafter, the processing different from that of the first embodiment will be mainly described.

After an ECG signal for a unit time is measured (step S100) and heart rate information is calculated (step S101), the ECG signal quality determination unit 23 evaluates the quality of the ECG signal for a unit time taken-in in step S100 (step S200). Any indicator may be used for the evaluation of the signal quality. An example of the evaluation index of the ECG signal will be described below.

• Number of zero crossings (ZC): A frequency at which the ECG signal crosses the baseline. For example, when the number of zero crossings is larger than 100 times per second, it is not considered likely to occur under normal baseline fluctuation, and thus the signal quality is determined to be poor.

• Relative power spectral density ratio (RSQI): A ratio between a power spectral density within a frequency region in which the energy of a P wave, a QRS wave, and a T wave, which are characteristic waveforms of the ECG signal, is concentrated and a power spectral density of the entire ECG signal. When the RSQI is less than a predetermined threshold value, the signal quality is determined to be poor.

On the other hand, after a PPG signal for a unit time is measured (step S110) and heart rate information is calculated (step S111), the PPG signal quality determination unit 33 evaluates the quality of the PPG signal for a unit time taken-in in step S110 (step S210). Any indicator may be used for the evaluation of the signal quality. An example of the evaluation index of the PPG signal will be described below.

• Perfusion Index (PI): A ratio of the AC (alternating current) component to the DC (direct current) component of the PPG signal. As the PI value decreases, the (SN) ratio becomes worse. For example, when the PI value of the PPG signal is less than 1, the signal quality is determined to be poor.

• Number of zero crossings (ZC): A frequency at which the sign of the AC component of the PPG signal changes. For example, when the number of zero crossings is larger than 10 times per second, it is not considered likely to occur under a normal heart rate, and thus the signal quality is determined to be poor.

• Relative Power Spectral Density Ratio (RSQI): A ratio between a power spectral density within a frequency region in which the energy of the waves during the systolic and diastolic phases of the heartbeat is concentrated and a power spectral density of the entire PPG signal. When the RSQI is less than a predetermined threshold value, the signal quality is determined to be poor. Since most of the energy of the waves during the systolic and diastolic phases is concentrated in the frequency region of 1 to 2.25 Hz, the RSQI may be calculated from the power spectral density (PSD) in this band and the power spectral density (PSD) of the entire signal (e.g., 0 to 8 Hz), as shown in the following equation, for example.

[Equation 1]

$$R_{SQI} = \sum_{f=1}^{2.25} PSD \Big/ \sum_{f=0}^{8} PSD$$

Note that the evaluation index of the signal quality of the ECG signal or the PPG signal is not limited to the above, and any index may be used. In addition, the signal quality may be determined to be good or poor by only one evaluation index, or the final signal quality may be determined to be good or poor by integrating the evaluation results of a plurality of evaluation indices.

The information regarding the presence or absence of a P wave is acquired from the time-series data of the ECG signal (step S102), as in the first embodiment. On the other hand, the acquisition source of the information regarding heart rate variability is dynamically switched depending on the signal quality of the ECG signal and the PPG signal. Specifically, when the signal quality of the ECG signal is determined to be good (step S201), the information regarding heart rate variability for a unit time is acquired from the time-series data of the ECG signal (step S202). When the signal quality of the ECG signal is determined to be poor (step S201), the information regarding heart rate variability for a unit time is acquired from the time-series data of the PPG signal (step S112). However, when the signal quality of the PPG signal is also determined to be poor (step S211), the data for a unit time is discarded and the process is terminated without performing the AF determination.

The subsequent processing (steps S120 and S121) is the same as that of the first embodiment.

According to the process of the present embodiment, the ECG signal is preferentially used during a period in which the quality of the ECG signal is good (that is, when body motion is slight), and the PPG signal is complementarily used during a period in which the quality of the ECG signal is poor (that is, when body motion is significant). Because the R wave peak of the ECG signal is sharper than the peak of the PPG signal, the heartbeat interval and the heart rate variability can be calculated more accurately (with smaller timing error) using the ECG signal. Therefore, the determination accuracy of atrial fibrillation can be improved relative to the first embodiment.

<Others>

The embodiments described above are merely illustrative of configuration examples of the present invention. The present invention is not limited to the specific aspects described above, and various modifications are possible within the scope of the technical idea of the present invention. For example, as the fixing member for fixing the electrodes 11 in a state in which the electrodes are pressed against the living body, a band-shaped (belt-shaped) member that is wrapped around the measurement portion of the living body in a state in which the electrodes 11 are brought into contact, as in the above embodiments, an envelope-shaped member that covers and wraps the measurement portion of the living body, or a ring-shaped member may be used. Additionally, the fixing member may have elasticity or deformability in order to correspond to the size (diameter) of the measurement portion of the living body. Alternatively, when the fixing member is made of a non-elastic material, the fixing member may have a structure that allows adjustment of the length. The number of electrodes 11 may be one or more. The arrangement of the plurality of electrodes 11 is not necessarily one line, and the electrodes 11 may be arranged in a two-dimensional array. In addition, the electrodes 11 may be arranged at irregular intervals, instead of at equal intervals. The electrode 11 may be integrated with the fixing member (band 10), or may have a separate structure from the fixing member. The control body 12 need not be fixed to the band 10. For example, the control body 12 and the band 10 may be formed as separate structures, and the control body 12 and the band 10 (the electrode 11) may be connected to each other by a cable. In addition, as the pulse wave sensor, a pressure pulse wave sensor may be used in addition to the PPG sensor.

The present specification includes the following disclosures.

[Supplementary Note 1]

A biological signal measurement device (1) configured to be worn by a user and to perform constant measurement of a biological signal, the biological signal measurement device (1) including:

an ECG sensor (14) configured to measure an ECG signal on a limb of the user;
a pulse wave sensor (15) configured to measure a pulse wave of the user; and an information processing unit (16) configured to acquire information regarding a heart rate variability and information regarding an abnormality of an electrocardiographic waveform, based on time-series data of the ECG signal obtained by the ECG sensor (14) and time-series data of the pulse wave obtained by the pulse wave sensor(15).

[Supplementary Note 2]

The biological signal measurement device (1) according to Supplementary Note 1, in which the information regarding the abnormality of the electrocardiographic waveform includes information regarding a presence or absence of a P wave, and

the information processing unit (16) detects an occurrence of atrial fibrillation, based on the information regarding the heart rate variability and the information regarding the presence or absence of the P wave that are acquired from the time-series data of the ECG signal and the time-series data of the pulse wave that are constantly measured.

[Supplementary Note 3]

The biological signal measurement device (1) according to Supplementary Note 2, in which the information processing unit (16) generates an index related to atrial fibrillation, based on a detection result of atrial fibrillation during a predetermined measurement period, and records or outputs the index.

[Supplementary Note 4]

The biological signal measurement device (1) according to Supplementary Note 3, in which the index related to atrial fibrillation includes at least one of a longest atrial fibrillation duration during the predetermined measurement period, the number of occurrences of atrial fibrillation during the predetermined measurement period, or a cumulative time of atrial fibrillation during the predetermined measurement period.

[Supplementary Note 5]

The biological signal measurement device (1) according to any one of Supplementary Notes 1 to 4, in which the information processing unit (16) acquires the information regarding the abnormality of the electrocardiographic waveform from the time-series data of the ECG signal, and acquires the information regarding the heart rate variability from the time-series data of the pulse wave.

[Supplementary Note 6]

The biological signal measurement device (1) according to any one of Supplementary Notes 1 to 5, in which the information processing unit (16) includes an ECG signal quality determination unit (23) configured to determine whether signal quality of the ECG signal is good or poor, and a pulse wave signal quality determination unit (33) configured to determine whether signal quality of the pulse wave is good or poor, and

acquires the information regarding the heart rate variability from the time-series data of the ECG signal during a period in which the signal quality of the ECG signal is determined to be good, and
acquires the information regarding the heart rate variability from the time-series data of the pulse wave during a period in which the signal quality of the ECG signal is determined to be poor.

[Supplementary Note 7]

The biological signal measurement device (1) according to any one of Supplementary Notes 1 to 6, including:

a dry electrode (11); and
a member (10) configured to fix the electrode (11) in a state in which the electrode (11) is pressed against the limb of the user, in which
the ECG sensor (14) measures the ECG signal by the electrode (11) fixed by the member (10).

[Supplementary Note 8]

The biological signal measurement device (1) according to any one of Supplementary Notes 1 to 7, in which the pulse wave sensor (15) is a photoplethysmography (PPG) sensor.

[Supplementary Note 9]

A method for controlling a biological signal measurement device (1) configured to be worn by a user and to perform constant measurement of a biological signal, the method including:

measuring an ECG signal on a limb of the user by an ECG sensor (14);
measuring a pulse wave of the user by a pulse wave sensor (15); and
acquiring information regarding a heart rate variability and information regarding an abnormality of an electrocardiographic waveform, based on time-series data of the ECG signal obtained by the ECG sensor (14) and time-series data of the pulse wave obtained by the pulse wave sensor (15).

REFERENCE SIGNS

1: Biological signal measurement device
10: Band
11: Electrode
12: Control body
13: Fixing mechanism
14: ECG sensor
15: Pulse wave sensor
16: Information processing unit

**Claims**

1. A biological signal measurement device configured to be worn by a user and to perform constant measurement of a biological signal, the biological signal measurement device comprising:

    an ECG sensor configured to measure an ECG signal on a limb of the user;
    a pulse wave sensor configured to measure a pulse wave of the user; and
    an information processing unit configured to acquire information regarding a heart rate variability and information regarding an abnormality of an electrocardiographic waveform, based on time-series data of the ECG signal obtained by the ECG sensor and time-series data of the pulse wave obtained by the pulse wave sensor.

2. The biological signal measurement device according to claim 1, wherein

    the information regarding the abnormality of the electrocardiographic waveform includes information regarding a presence or absence of a P wave, and
    the information processing unit detects an occurrence of atrial fibrillation, based on the information regarding the heart rate variability and the information regarding the presence or absence of the P wave that are acquired from the time-series data of the ECG signal and the time-series data of the pulse wave that are constantly measured.

3. The biological signal measurement device according to claim 2, wherein
the information processing unit generates an index related to atrial fibrillation, based on a detection result of atrial fibrillation during a predetermined measurement period, and records or outputs the index.

4. The biological signal measurement device according to claim 3, wherein
the index related to atrial fibrillation includes at least one of a longest atrial fibrillation duration during the predetermined measurement period, the number of occurrences of atrial fibrillation during the predetermined measurement period, or a cumulative time of atrial fibrillation during the predetermined measurement period.

5. The biological signal measurement device according to any one of claims 1 to 4, wherein
the information processing unit acquires the information regarding the abnormality of the electrocardiographic waveform from the time-series data of the ECG signal, and acquires the information regarding the heart rate variability from the time-series data of the pulse wave.

6. The biological signal measurement device according to any one of claims 1 to 5, wherein

    the information processing unit
    includes an ECG signal quality determination unit configured to determine whether signal quality of the ECG signal is good or poor, and a pulse wave signal quality determination unit configured to determine whether signal quality of the pulse wave is good or poor, and
    acquires the information regarding the heart rate variability from the time-series data of the ECG signal during a period in which the signal quality of the ECG signal is determined to be good, and

acquires the information regarding the heart rate variability from the time-series data of the pulse wave during a period in which the signal quality of the ECG signal is determined to be poor.

7. The biological signal measurement device according to any one of claims 1 to 6, comprising:

   a dry electrode; and
   a member configured to fix the electrode in a state in which the electrode is pressed against the limb of the user, wherein
   the ECG sensor measures the ECG signal by the electrode fixed by the member.

8. The biological signal measurement device according to any one of claims 1 to 7, wherein
   the pulse wave sensor is a photoplethysmography (PPG) sensor.

9. A method for controlling a biological signal measurement device configured to be worn by a user and to perform constant measurement of a biological signal, the method comprising:

   measuring an ECG signal on a limb of the user by an ECG sensor;
   measuring a pulse wave of the user by a pulse wave sensor; and
   acquiring information regarding a heart rate variability and information regarding an abnormality of an electrocardiographic waveform, based on time-series data of the ECG signal obtained by the ECG sensor and time-series data of the pulse wave obtained by the pulse wave sensor.

**FIG. 1**

AT REST
(WITH NO BODY MOVEMENT)

ACTIVE
(WITH BODY MOVEMENT)

ECG SENSOR

PULSE WAVE
SENSOR

INFORMATION REGARDING PRESENCE OR ABSENCE OF P WAVE
INFORMATION REGARDING HEART RATE VARIABILITY

CONSTANT MONITORING OF ATRIAL FIBRILLATION
RECORDING OF AF burden

EP 4 659 673 A1

*FIG. 2*

*FIG. 3*

FIG. 4

EP 4 659 673 A1

*FIG. 5*

```
                    ┌─────────────┐
                    │   START     │
                    └──────┬──────┘
           ┌───────────────┴───────────────┐
           ▼                               ▼
  ┌──────────────────┐ ⌐S100   ┌──────────────────┐ ⌐S110
  │ MEASURE ECG SIGNALS│        │ MEASURE PPG SIGNAL │
  └────────┬─────────┘          └────────┬─────────┘
           ▼                             ▼
  ┌──────────────────┐ ⌐S101   ┌──────────────────┐ ⌐S111
  │ CALCULATE HEART RATE│       │ CALCULATE HEART RATE│
  │   INFORMATION     │         │   INFORMATION     │
  └────────┬─────────┘          └────────┬─────────┘
           ▼                             ▼
  ┌──────────────────┐ ⌐S102   ┌──────────────────┐ ⌐S112
  │ DETERMINE PRESENCE OR│      │ DETERMINE HEART RATE│
  │ ABSENCE OF P WAVE FROM│     │ VARIABILITY FROM PPG│
  │   ECG SIGNAL     │          │     SIGNAL        │
  └────────┬─────────┘          └────────┬─────────┘
           └───────────────┬─────────────┘
                           ▼
                  ┌──────────────────┐ ⌐S120
                  │  AF DETERMINATION │
                  └────────┬─────────┘
                           ▼
                  ┌──────────────────┐ ⌐S121
                  │ RECORD AF INFORMATION│
                  └────────┬─────────┘
                           ▼
                    ┌─────────────┐
                    │    END      │
                    └─────────────┘
```

FIG. 6

ELECTRODE PAIR #1

ELECTRODE PAIR #2

ELECTRODE PAIR #3

EP 4 659 673 A1

FIG. 7

*FIG. 8*

*FIG. 9*

FIG. 10

## FIG. 11

START

MEASURE ECG SIGNALS — S100

CALCULATE HEART RATE INFORMATION — S101

EVALUATE QUALITY OF ECG SIGNAL — S200

DETERMINE PRESENCE OR ABSENCE OF P WAVE FROM ECG SIGNAL — S102

QUALITY OF ECG SIGNAL — S201
POOR
GOOD

DETERMINE HEART RATE VARIABILITY FROM ECG SIGNAL — S202

MEASURE PPG SIGNAL — S110

CALCULATE HEART RATE INFORMATION — S111

EVALUATE QUALITY OF PPG SIGNAL — S210

QUALITY OF PPG SIGNAL — S211
POOR
GOOD

DETERMINE HEART RATE VARIABILITY FROM PPG SIGNAL
S112

AF DETERMINATION — S120

RECORD AF INFORMATION — S121

END

## INTERNATIONAL SEARCH REPORT

| International application No. |
| --- |
| **PCT/JP2024/006329** |

**A.  CLASSIFICATION OF SUBJECT MATTER**

*A61B 5/33*(2021.01)i; *A61B 5/02*(2006.01)i; *A61B 5/352*(2021.01)i; *A61B 5/353*(2021.01)i; *A61B 5/361*(2021.01)i
FI:    A61B5/33 200; A61B5/02 310A; A61B5/352 100; A61B5/353; A61B5/361

According to International Patent Classification (IPC) or to both national classification and IPC

**B.  FIELDS SEARCHED**

Minimum documentation searched (classification system followed by classification symbols)

A61B5/33; A61B5/02; A61B5/352; A61B5/353; A61B5/361

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
| --- | --- | --- |
| X | JP 2023-035439 A (OMRON HEALTHCARE CO., LTD.) 13 March 2023 (2023-03-13) abstract, paragraphs [0008], [0031]-[0032], [0053], [0056], [0060], fig. 1, 5, 9A, 10 | 1, 5-9 |
| A | | 2-4 |
| A | JP 2016-185288 A (SHARP KABUSHIKI KAISHA) 27 October 2016 (2016-10-27) paragraphs [0032]-[0046], fig. 1-2G | 2-4 |
| A | JP 2016-064125 A (SHINANO KENSHI KABUSHIKI KAISHA) 28 April 2016 (2016-04-28) paragraphs [0062]-[0063], fig. 23 | 1-9 |
| A | JP 2016-214641 A (SEIKO EPSON CORPORATION) 22 December 2016 (2016-12-22) paragraphs [0039]-[0074], fig. 1-2 | 1-9 |
| A | WO 2013/161729 A1 (MURATA MANUFACTURING CO., LTD.) 31 October 2013 (2013-10-31) paragraphs [0047]-[0071], fig. 1-3 | 1-9 |

☐ Further documents are listed in the continuation of Box C.          ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **30 April 2024** | **14 May 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

### INTERNATIONAL SEARCH REPORT
Information on patent family members

International application No.

**PCT/JP2024/006329**

| Patent document cited in search report | | | Publication date (day/month/year) | Patent family member(s) | | | Publication date (day/month/year) |
|---|---|---|---|---|---|---|---|
| JP | 2023-035439 | A | 13 March 2023 | WO | 2023/032760 | A1 | |
| JP | 2016-185288 | A | 27 October 2016 | (Family: none) | | | |
| JP | 2016-064125 | A | 28 April 2016 | US | 2017/0277858 | A1 | |
| | | | | paragraphs [0181]-[0185], fig. 23 | | | |
| | | | | WO | 2016/043299 | A1 | |
| | | | | EP | 3196836 | A1 | |
| JP | 2016-214641 | A | 22 December 2016 | US | 2016/0338598 | A1 | |
| | | | | paragraphs [0060]-[0124], fig. 1-2 | | | |
| WO | 2013/161729 | A1 | 31 October 2013 | US | 2015/0038808 | A1 | |
| | | | | paragraphs [0064]-[0088], fig. 1-3 | | | |
| | | | | EP | 2842483 | A1 | |

Form PCT/ISA/210 (patent family annex) (July 2022)

**REFERENCES CITED IN THE DESCRIPTION**

**Patent documents cited in the description**

- JP 2004016248 A